# EUROPEAN PATENT APPLICATION

(11) **EP 4 389 042 A1**
(43) Date of publication of application: **26.06.2024**
(21) Application number: 23216791.6
(22) Date of filing: 14.12.2023
(51) Int. Cl.: A61B 18/12, A61B 18/00

(54) **ELECTROSURGICAL GENERATOR**

(30) Priority: 23.12.2022 US 202263434989 P
(71) Applicant: Olympus Winter & Ibe GmbH, 22045 Hamburg (DE)
(72) Inventor: Ramin, Daniel, 14558 Nuthetal (DE); Fähsing, Thomas, 12305 Berlin (DE); Dietrich, Stefan, 14469 Potsdam (DE)
(74) Representative: Glawe, Delfs, Moll

(57) **Abstract**

Electrosurgical generator comprising an inverter unit (27) generating HF energy and a high-voltage connection supplying the HF energy to an output socket (17) for an electrosurgical instrument (19). The high-voltage connection comprises a base module (2) with a distribution unit and at least one connection port. The distribution unit comprises a HF distribution line (4) and data communication wiring (5), both being supplied to the at least one connection port for which a sub module (7) is provided connecting to the HF and the data signals and supplying the output socket (17). Thereby electrical connections and data communications are established at once. Consequently, inserting a different sub module allows for simplified alteration of type and variant of the generator. A newly developed version of the sub module may just be inserted into the connection port without any need of alteration of the base module. Modernization and updating is facilitated.

## Description

The invention relates to an electrosurgical generator configured to output a high-frequency alternating voltage to an electrosurgical instrument. The electrosurgical generator comprises a casing, a power supply unit, an inverter unit for high-voltage that generates a high-frequency alternating voltage to be supplied to at least one output socket configured for connection of the electrosurgical instrument.

In electrosurgery or high-frequency surgery, an electrosurgical instrument such as an electroscalpel is used to apply high-frequency alternating current to tissue in the human body. Usually high-frequency in the radiofrequency range of about 200 kHz to up to 4,000 kHz is used. This results in local heating of the tissue. Thereby, the tissue is cut or severed by heating, and the tissue is removed by thermal resection. A major advantage of this is that bleeding can be stopped at the same time as the cut by closing the affected vessels, and electrosurgical instruments can be used for other applications, such as coagulation. Electrosurgical generators are utilized to supply power to electrosurgical instruments and are designed to deliver a high-frequency alternating voltage to an electrosurgical instrument, so they are medical devices.

In order to provide electrosurgical generators for various fields of medicine, different types of electrosurgical generators are required having different components and featuring different functionality. There are various types of electrosurgical generators: apart from a type for general surgery various types exist, e.g., specialized for urology, gynecology, gastroenterology, pulmonology, ENT (ear, nose, throat), phlebology and visceral surgery just to name a few. Within each of these types there are also different variants of electrosurgical generators, some having lesser and others having a bigger range of functionality and power. This is beneficial for the surgeon as to having the right electrosurgical generator for a specific surgery task. At the other hand, this means a huge number of different varieties of electrosurgical generators.

Developing and manufacturing of such a huge variety of electrosurgical generators is cumbersome for the manufacturer. Moreover, such a big variety of the surgical electrosurgical generator makes modernizing more complicated thus hinders innovations.

It is thus an object of the invention to provide an electrosurgical generator that reduces this drawback.

The solution according to the invention consists in an electrosurgical generator according to the features of the independent claim. Advantageous developments are the subject matter of the dependent claims.

In an electrosurgical generator configured to output HF energy to an electrosurgical instrument, comprising an inverter unit generating the HF energy to be output to the electrosurgical instrument, and a high-voltage connection supplying the HF energy via at least two electrode lines to at least one output socket configured for connection of the electrosurgical instrument, according to the invention the high-voltage connection comprises a base module (2) which is provided with a distribution unit and at least one connection port, the distribution unit comprising a HF energy distribution line and a data communication wiring for data signals, and being configured to distribute the HF energy supplied from the inverter unit as well as the data signals to the at least one connection port, said at least one connection port being configured to be connected to a sub module and conveying the HF energy and the data signals to said sub module which supplies the output socket with the HF energy.

In the following, some expressions that are used within the context of the invention are explained:
The inverter unit is a device to provide the actual high-frequency output for the surgical instrument to be connected to the output socket. The term inverter is rather broad and comprises actual inverter technology as well as converters, e.g., forward or flyback converters, or amplifiers.

In the context of the present patent the term "HF" means "high-frequency" and relates to frequencies in the radiofrequency range of 200 kHz to 4000 kHz as generated by the inverter unit of the electrosurgical generator. However, for electrosurgical generators being capable of driving ultrasound instruments, the term "high-frequency" also relates to the ultrasound frequency range, typically in the range of 20 kHz to 200 kHz (ultrasound surgical generators). The HF energy may be output as high frequency alternating voltage which is considered as being high voltage in the context of the present patent if having amplitudes in the high voltage range, in particular up to 10 kV, preferably up to 4000 Volt and further preferably more than 100 Volt.

A "connection port" is a device where the sub module can be connected to the base module and its distribution unit such that electrical contacting is made. The connection port may be connected to the related sub module via a single (e.g., a hybrid connector), a pair or several connectors.

The term "hybrid connector" is to be understood as a connector comprising different sections for conveying HF power and signals. The hybrid connector features a section for HF power and another section for signals, and those sections are combined into a single connector.

In the context of the present patent, a "sub module" is a module to be attached to the base module and being configured to supply the output socket with the high-frequency alternating voltage as generated by the inverter unit. Typically the sub module comprises a printed circuit board. The output socket may be arranged directly on the sub module or connected to it via a connection.

The invention draws on the fact that by providing a base module as defined in the independent claim, the connection port or connection ports of the base module can be connected to various configurations of sub modules selected for a specific type or variant of the electrosurgical generator. Thereby, forming the electrosurgical generator to the needs of a specific type or variant at hand is greatly facilitated. Various types or variants could thereby be manufactured without any further modification other than inserting the relevant sub module(s) into the appropriate connection port in order to achieve the desired functionality. Thereby electrical connections as well as data communications are established at once. As a result, by inserting a different sub module in one or another connection port allows for a much simplified alteration of the type and variant of the electrosurgical generator. Manufacturing of many different types and variants of electrosurgical generators is thus possible with much less effort.

Moreover, even modernization and updating is facilitated. If a new version of the sub module is developed than it may be just inserted into the connection port without any need of alteration of the base module. The base module provides for the proper supply of the sub module in any case.

Preferably, the at least one connection port is configured as a universal connector to be connectable to sub modules of different types, in particular differing by number and/or kind of electrode lines. This yields the advantage that an existing type or variant of the electrosurgical generator could be easily modified or reconfigured by changing any of the sub modules to another sub module, in particular another sub module having a different functionality and/or requiring a different connection to the high-frequency distribution unit. Preferably, the different types of the sub modules comprise a monopolar sub module, a bipolar sub module, a neutral electrode sub module and/or a universal sub module. It is a significant advantage that thereby different kinds of sub module, like monopolar and bipolar, can be used and if necessary exchanged without any alteration of the base module. The connection port provides the necessary electrodes and data signaling, thereby giving optimal flexibility in terms of employing sub modules of different types. Prominent examples for such different types of sub modules are a monopolar socket module, a bipolar socket module, a neutral electrode socket module and/or a universal socket module. This is not limiting, other types of optional sub modules may be provided.

Advantageously, the distribution unit distributes the HF energy and the data signals to a plurality of connection ports. Although the invention works with just having a single connection port, its utility increases if a plurality of connection ports are to be served by the distribution unit. This provides for the possibility of creating a rather high number of variants by using different sub modules in the various connection ports.

Preferably, the at least one connection port is formed as a hybrid connector comprising a first section for HF energy and the second section for of the data signals. This means by plugging in of the hybrid connector contacts for proper supply with both, the HF energy as well as signals for data communication, will be established at once. The sections may be separated by walls made of preferably isolating material and/or an interspace of sufficient dimensions for proper isolation. There may be additional sections, e.g., an isolating or spacing section and/or a section for other types of electrical contacts, e.g., for a low voltage power supply.

Advantageously, the HF distribution line comprises high voltage conductors for active electrode, a neutral electrode and at least an additional electrode, preferably a second active electrode. By virtue of such a combination of electrodes various different kinds of sub modules, in particular monopolar socket modules as well as bipolar socket module, can be supplied with HF voltage. Further, the additional electrode that may be used as a second active electrode for special electrosurgical instruments or for powering an additional sub module supplying a second electrosurgical instrument. Preferably, the conductor of the additional electrode is switchable between various states, like acting as a second active electrode or being connected with one of the other electrodes, the neutral or the active electrode. By such a switchable characteristic versatility of the additional electrode is increased.

In a preferred embodiment, the HF distribution line further comprises a conductor for voltage in the ultrasonic frequency range (ultrasonic frequency voltage). Thereby, it is further enabled to supply sub modules for ultrasound instrument or sub modules for combined ultrasound and HF instruments (e.g., Thunderbeat of Olympus Surgical). Hereby, the conductor for ultrasonic frequency voltage is preferably an additional conductor, but may alternatively configured as a shared conductor. By "shared conductor" it is meant that said conductor is a switchable conductor, namely switchable between being an ultrasound conductor or a conductor for another electrode, e.g., a conductor for a second active electrode. Thereby a high degree of flexibility is provided to use the additional conductor for ultrasound, e.g., if a sub module requiring ultrasound frequency voltage is present, or for a second active electrode, e.g., if a second electrosurgical instrument shall be powered.

The base module preferably further comprises an appliance voltage distribution wiring configured to convey operating power for the sub module via the connection port. By virtue of this, electrical power necessary for powering appliances, like internal circuitry of the sub modules, can be conveyed using the same connection port. Thereby even powered "smart" sub modules having a lot of circuitry on board can be connected via the connection port as well as just passive modules that do not require power alimentation. This is particularly beneficial if this is embodied as a third section of the hybrid connector for maximum ease of handling.

In a preferred embodiment, the base module is a circuit board, and the HF distribution line is configured as a high-voltage bus bar. By configuring the base module as a circuit board a solid mechanical base is formed which is enabled to provide proper support for the connection ports, preferably the hybrid connectors. Thereby, plugging of the sub modules to the connection port, preferably hybrid connectors, is much facilitated and the sub modules can be mechanically supported by said circuit board in the plugged-in state, too. However, this is not a must and the base module can also be configured as a cable set comprising the connection ports and conveying the HF energy, the data communication and if applicable the appliance voltage.

By such a bus bar the HF energy can be distributed efficiently from a central feeding point to the plurality of connection ports. Moreover, the connection ports, particularly the hybrid connectors, can be located at any point along the bus bar, which further increases flexibility of positioning of the connection ports, particularly the hybrid connectors.

Advantageously, the high-voltage bus bar is formed by a plurality of, preferably at least three, elongated conductors having a defined spacing between them. Thereby, proper spacing can be ensured as it is required for insulation of the conductors, which is of particular importance if high-voltage in the range of several hundred volts or more is to be used for the HF energy. Moreover, such a configuration of the conductors allows for arrangement of the sub modules side by side along a line as defined by the orientation of the conductors. This provides for a well arranged and clearly laid out positioning of the sub modules, even in the case of complex electrosurgical generators having a plurality of the sub modules. Preferably, the high-voltage bus bar is formed by rails or stripes on a circuit board. Thereby rail like elongated conductors can be formed directly on the circuit board in a simple and reliable manner. Any risk of dislocation of the conductors or unwanted variations in their relative spacing is effectively eliminated if the conductors are formed as stripes on the circuit board, preferably a circuit board of the base module.

In an embodiment, the output socket may be arranged directly on the sub module. This provides for a short supply path from the sub module to the output socket. In an alternative variant, the output socket may be detached from the sub module. In this variant, the sub module is connected to the output socket by an interconnection line. Such an interconnection line allows for flexible positioning of the output socket, e.g., it can be located anywhere on the casing of the electrosurgical generator without regard to the actual location of the sub module positioned on the base module. Said interconnection line preferably comprises HF energy and signal leads. By providing such additional signal leads which are preferably integrated into a common cable with the HF energy leads, rather complex electrosurgical instruments and those instruments having a hand switch can be plugged into the output socket. The signal leads are preferably connected by the sub module to the data communication wiring of the base module.

Preferably, relays are provided for the connection ports, each of the relays interacting with one of the connection ports and being configured to switch on or to block the HF energy conveyed by said one of the connection ports. By virtue of this a protection effect can be achieved. In particular, if the sub module is properly connected at the connection port to the base module and when the instrument plugged into the output socket shall be energized, then the relays will switch on and thereby will allow the HF energy to be passed from the distribution unit of the base module via the sub module to instrument plugged into the output socket. By virtue of this, if the sub module is not seated properly in the connection port or when there is no actual demand due to not activated instrument then the relay will not be activated and no HF energy flows to the instrument. Thereby, an additional safety feature can be achieved.

Advantageously, the relay interacting with one of the connection ports is configured to switch on dependent on a signal issued if the sub module has passed a compatibility check. Thereby it can be achieved that HF energy is only switched on by the relay if the correct sub module is mounted. If the sub-module is not the correct then the relay will not switch on thereby blocking the HF energy. This is a considerable increase in device safety.

The relay may be located at the connection port or in the vicinity of the connection port on the base module. However, in a preferred embodiment the relay is located on the sub module, and preferably the relay is energized by an individual enable signal conveyed by said one of the connection ports connected to said sub module. The relay is thus placed on the sub module which it shall protect, and therefore it can be configured according to the requirements of the respective sub modul. This allows for a relay configuration being suited better to the various requirements of the different sub modules, as opposed to a kind of universal relay which would be necessary if it was located on the base module.

In a preferred embodiment which may deserve independent protection on its own, an optional extension board is provided which comprises at least one additional connection port and an extended HF distribution line and an extended data communication distribution wiring, wherein the at least one additional connection port is connected to said distribution line and to the data communication distribution wiring like the connection port of the base module.

By virtue of such an extension module additional connection ports can be provided, thereby enabling the electrosurgical generator to offer a plurality of additional sub modules on the extension module. This allows for an accommodation of the hardware of even highly complex electrosurgical generators while still employing the same base module and just enhancing it by said extension module. The usage of different parts for "lean" or "rich" module and output socket configurations of the electrosurgical generators can be minimized.

In a preferred embodiment, the base module comprises an interface for attaching the extension module, such that the HF distribution line of the base module is/are connected to the HF distribution line of the extension module, and the data distribution wiring and/or appliance voltage distribution wiring of the base module is/are connected to respective distribution wirings of the extension module board. By virtue of the interface, the extension module is connected to the base module acting as an expansion of the distribution unit. Due to this, supply of the additional sub modules to be placed on the extension module is facilitated, allowing for an easy population with additional sub modules for increased functionality of the electrosurgical generator.

Advantageously, the distribution unit of the extension module is provided with an supplemental conductor for another electrode. By virtue of this, the sub modules placed on the extension module may use a different active electrode than the sub modules placed on the base module. This allows for an increased variety of different sub modules, and further increases the range of interoperability. For example it is feasible thereby to employ monopolar sub modules in a bridged configuration, whereas one sub module for positive side is placed on the base module and the complementary monopolar sub module for a negative side is to be placed on the extension module which is configured to employ the additional conductor as another active electrode, preferably having the converse, i.e. negative, polarity. By virtue of this, rather sophisticated configurations of electrodes can be achieved while sticking to the inventive concept of having a base module and an extension module.

Preferably, the interface comprises at least one switch configured to isolate or to connect the supplemental conductor of the extension module to one of the conductors of the base module, preferably the neutral electrode conductor of the base module. Thereby, the supplemental conductor of the extension module can be selectively used as an additional electrode having its own independent potential or as a neutral electrode. In the latter case, two monopolar sub modules in a monopolar bridged configuration can be supplied, one being on the base module and the other being on the extension module.

Advantageously, the interface further comprises at least one changeover switch configured to cross-connect electrode conductors of the base module to those of the extension module, preferably being actuated automatically depending on the type of the sub module connected to the at least one connection port of the extension module. By virtue of such cross-connection the extension module is enabled to supply its sub modules with a different potential as the one used on the base module for the active electrode. This allows in particular for electrosurgical instruments being supplied in a dual bipolar manner. Further, the connection of the interface and its conductors to the extension module will be selected automatically according to the specifications of the sub module that is actually inserted in the connection port of the extension module. This increases the range of adaptability and flexibility in terms of types of sub modules that can be used, without requiring manual interaction for proper configuration.

Preferably, the extension module has an own HF energy input terminal and the base module is supplied with the HF energy via the interface. This allows for a direct supply of the extension module with the HF energy, and further allows for a retrograde supply of the base module from the extension module via the interface. This ensures short distances for optimum supply of high voltage, and further has the advantage that an additional conductor present on the extension module can be supplied directly with the required HF energy.

The invention is explained in more detail below by way of examples in conjunction with the accompanying drawings, showing advantageous embodiments. In the figures:
- Fig. 1: shows frontal view of an electrosurgical generator with an attached electrosurgical instrument;
- Fig. 2a, b: show schematically perspective views of two electrosurgical generators according to a first and a second exemplary embodiment;
- Fig. 3: shows a front view of a connection port embodied as a hybrid connector;
- Fig. 4: shows a schematic functional diagram of the base module of the electrosurgical generator according to the exemplary embodiments; and
- Fig. 5: shows a schematic functional diagram of the base module of Fig. 4 in addition with an extension module in a first configuration;
- Fig. 6: shows a schematic functional diagram of the base module of Fig.4 in addition with the extension module in a second configuration;
- Fig. 7: shows a schematic functional diagram of the base module of Fig.4 in addition with the extension module in a third configuration; and
- Fig. 8: shows a schematic view of a variant of the base module.

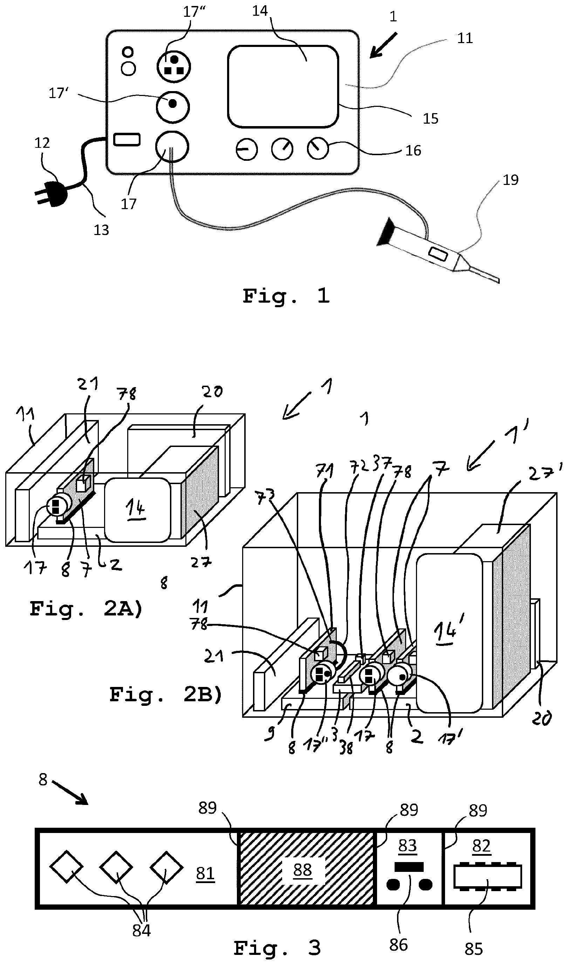

An electrosurgical generator according to an exemplary embodiment of the invention is illustrated in Fig. 1. The electrosurgical generator, identified as a whole by reference numeral 1, comprises a housing 11 having an output socket 17 for connection of an electrosurgical instrument 19. A power supply cable 13 is provided with a plug 12 for connection to an electrical power source (not shown) which may be an electricity grid, like the AC mains in a building, or an off-grid source of electric energy, like a 12 Volt or 24 Volt battery in a vehicle or mobile hospital. Further, a user interface 14 is provided comprising a display 15 which may be a touchscreen, or knobs 16 may be present for inputs by a user.

Said electrosurgical instrument 19 comprises a cable which is to be plugged-in into the output socket 17 in order to supply HF energy to the electrosurgical instrument 19 which in the depicted exemplary embodiment is an electroscalpel.

The electrosurgical generator 1 may or may not have additional sockets 17' and 17" depending on the type or variant of the electrosurgical generator 1.

Fig. 2A, B show schematically perspective views of electrosurgical generators according to a first exemplary embodiment 1 and an electrosurgical generator 1' according to a second exemplary embodiment. Both embodiments share the same basic concept that differ from each other in number and kind of modules which are installed. Both exemplary embodiments comprise a power supply unit 21 that is fed by electrical energy from the power supply by the power supply cable 13. The power supply unit 21 provides electrical power, usually DC power, to the various components units and modules of the electrosurgical generator 1. In particular, it provides electrical power to an inverter unit 27 configured for generating the HF energy by producing high-frequency alternating voltage, usually in the high-voltage range of a few kilovolts, but may have amplitudes in a range of several 10 Volt up to 4000 Volt.

The kind of inverter unit 27 is selectable by the person skilled in the art since there are several concepts known in the art, for example a forward converter or a multilevel inverter. The key point is that the inverter unit 27 generates the high-frequency alternating voltage in a voltage range high enough to produce HF energy for proper operation of the electrosurgical instrument 19. Operation of the inverter unit 27 is governed by a control unit 20 which in turn is connected with the user interface 14 such that the user can issue directions and commands for operation of the electrosurgical generator, the control unit 20 generates corresponding control signals and governs the relevant components, units and modules of the electrosurgical generator 1 according to these instructions and commands. This is generally known in the art and therefore will not be described in more detail.

The HF energy generated by the inverter unit 27 is applied via an output connection to output socket 17. To this end, in in the first and second exemplary embodiment a base module 2 is provided which comprises a high-frequency distribution unit that is configured to convey the output voltage of the inverter unit 27 via a sub module 7 to the output socket 17, wherein the sub module 7 is placed in a connection port formed as an hybrid connector 8 (s. Fig. 3) on the base module 2. In the embodiment depicted in Fig. 2A) the sub module 7 carries the output socket 17 directly.

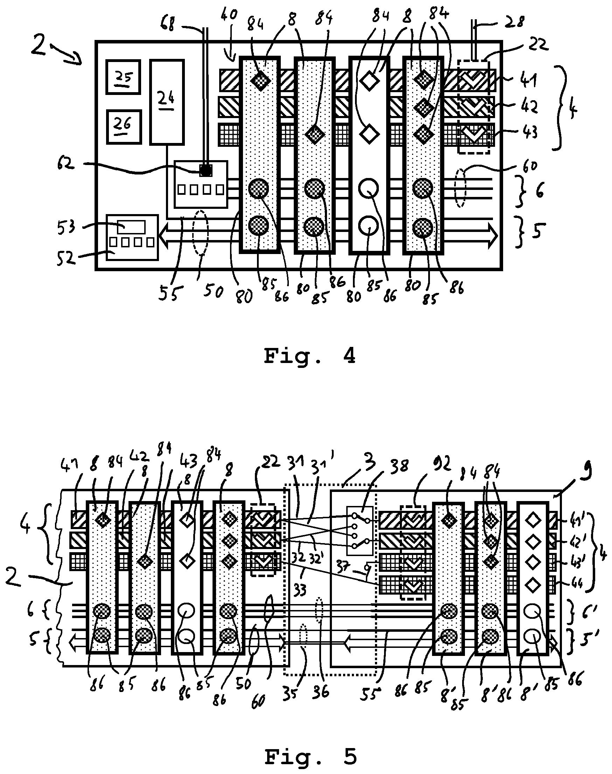

The second exemplary embodiment 1' shown in Fig. 2B) is similar and differs in particular in that two more sockets 17' and 17" are provided, each of which being provided with a respective own sub module 7. Two of the sub modules 7 are placed into respective connection ports formed as hybrid connectors 8 (s. Fig. 4) on the base module 2, whereas a third sub module 7 supplying the output socket 17" is placed into a corresponding connection port formed as an hybrid connector 8' (s. Fig. 5) on an extension module 9 that is an additional circuit board connected to the base module 2 via an interface 3.

The third sub module 7 is different in that its output socket 17" is positioned detached from the sub module 7. An interconnection cable 72 is provided which is a high voltage cable having integrated additional signal leads 73 and connects the detached output socket 17" to a printed circuit board 71 of said sub module 7. Thereby, the location of the detached output socket 17" is not strictly determined by the location of its corresponding sub module on the extension board 9, rather the detached output socket 17" can be nearly freely positioned, e.g., at a convenient location at the casing 11. Further, the second exemplary embodiment as depicted in Fig. 2B) differs in that in order to provide sufficient high-frequency power and control authority, a larger and more powerful inverter unit 27' is provided as well as a more sophisticated user interface 14'.

Configuration of the base module 2 will be explained next taking reference to Fig. 4. The base module 2 is provided with a distribution unit comprising a HF energy distribution line 4, a data communication distribution wiring 5 and an appliance voltage distribution wiring 6.

The HF distribution line 4 comprises a bus bar 40 comprising three stripe conductors configured for high-frequency and high-voltage, a first conductor 41 being for an active electrode 41, a second conductor being configured as neutral electrode 42, and a third conductor 43 being configured as a second active electrode in the depicted exemplary embodiment. The stripe conductors are dimensioned relatively wide and are separated from each other by a spacing 45 sufficient for isolation as is required for medical devices (usually demanded by respective national regulation. HF energy as generated by the inverter unit 27 is supplied to the high-frequency distribution unit 4 of the base module 2 via a HF energy supply cable 28 to a HF input terminal 22 which is located close to an edge of the base module 2 across all conductors 41, 42, 43 of the bus bar 40. By virtue of this, the high-frequency power as generated by the inverter unit 27 is directly supplied to the high-frequency distribution unit 4 of the base module 2.

Substantially parallel to the stripe conductors of the bus bar 40 an appliance voltage distribution wiring 6 is located on the base module 2. This appliance voltage distribution wiring 6 comprises a set of smaller conductors 46 arranged in parallel as a power supply bus bar 60, these conductors being configured for conducting power in order to operate appliance circuitry on the respective sub modules 7, like a 12 V DC supply and 5 V electronic power. These voltages are supplied from the power supply 21 and are conveyed to the appliance voltage distribution wiring 6 by means of a cable 68 and an appliance voltage supply terminal 62, to which the conductors of the appliance voltage distribution unit 6 are connected either directly or by a DC/DC converter.

Further, the data communication wiring 5 provided on the base module 2 comprises a data bus bar 50 having various lines running parallel to the HF_energy distribution line 4 and the appliance voltage distribution wiring 6. It is connected to a data terminal 52 which is connected to the operational control unit 20. In the exemplary embodiment the data bus is configured as being a CAN (control area network) bus, preferably having a rather low communication speed (CAN-Low), whereas the connection to the control unit 20 features a high communication speed (CAN-High). To this end, the terminal 52 may optionally be provided with a High/Low communication converter 53.

Further, in the embodiment depicted in Fig. 4, the base module 2 is further equipped with an optional device, namely a microprocessor 24 configured to control operation of the base module 2.

Now turning to a key aspect of the invention, a plurality of connection ports the are provided on the base module 2. Each of these comprises an optional guide 80 and a contact set 84 to the HF energy distribution line 4, a contact set 85 to the data communication wiring 5 and a contact set 86 to the appliance voltage distribution wiring 6. The optional guide 80 is configured to mechanically receive the respective sub module 7, wherein said sub module 7 in its inserted state is electrically connected to the HF energy distribution line 4, to the data communication wiring 5 and to the application voltage distribution wiring 6 via said connection ports and its contact sets 84, 85 and 86, respectively. A preferred embodiment of the connection port is a hybrid connector 8 as it is shown in Fig. 3. It is a single connector that comprises several sections, namely a HF energy section 81 for the contact set 84, a data section 82 for the contact set 85 which may be embodied as a CAN plug, and an auxiliary voltage section 83 for the contact set 86 which may comprise 12 Volt, 5 Volt and Ground contacts. The various sections 81, 82, 83 are preferably delimited from each other by internal walls 89. Further preferably, within the hybrid connector 8 a spacing section 88 is provided. It serves the purpose of creating an additional isolation of the HF energy section 81 and the high voltage at its contact set 84 from the sections 85 and 86 having the contact sets 82, 83 for the delicate low voltage and data signals. The spacing section 88 may be a hollow space using air as isolator or it may be filled by non-conducting material for additional isolation. The hybrid connector 8 further features an optional guide 80. The hybrid connector 8 has the advantage, that all the connections are performed automatically upon inserting the respective sub module 7 in the respective hybrid connectors 8.

The hybrid connectors 8 are configured identically, each of the hybrid connectors being capable of receiving any of the sub modules. To this end, each of the hybrid connectors 8 provides all of the contact sets 84, 85 and 86, however which contact set of which contact of the respective contact sets is or are actually used depends on the type of sub module 7 to be inserted. Thereby great flexibility results in terms of utilizing same or different sub modules 7. This flexibility can be even further enhanced by an optional extension module 9. It is configured similar to the base module 2 in that it comprises the same distribution lines and wirings like the base module 2 and further provides at least one additional hybrid connector 8' being connected to said distribution units. For connection of the extension module 9 to the base module 2 an interface 3 is provided. It is configured for attaching the extension board 9 to the base module 2 such that distribution units 4', 5', 6' of the base module 2 are connected to likewise distribution units 4, 5, 6, of the extension module 9.

By virtue of this, additional hybrid connectors 8' are provided so that more sub modules 7 can be utilized. Further, certain enhanced functionalities are achieved thereby which will be highlighted in the following.

Reference is now made to Fig. 5 showing a schematic functional diagram of the base module of Fig. 3 in addition with an extension module 9 according to a first configuration. Only a portion of the base module 2 is shown on the left-hand side of the figure carrying the distribution lines and wirings 4, 5, 6. On the right-hand side of the figure the extension module 9 is shown, and in the middle of the figure the interface 3 is shown connecting the extension module 9 to the base module 2. The distribution unit 4 of the extension module comprises three stripe conductors 41', 42', 43' as the base module 2 and further provides an supplemental stripe conductor 44. Further, hybrid connectors 8' are provided, in the example shown there are three additional hybrid connectors 8' on the extension module 9. They are configured like the hybrid connectors 8 on the base module 2 having contact sets 84, 85, 86including an additional contact 84' in the contact set 84 to the supplemental conductor 44.

The interface 3 comprises a changeover switch 38 which is configured as a double single-pole-double-throw (SPDT) relay. It is connected to the stripe conductors 41, 42, 43 of the base module 2 by interface lines 31, 31', 32, 32' and 33. Line 33 begins at the stripe conductor 43 of the base module 2 and is connected directly to the stripe conductor 44 of the extension module 9. Line 31 and 31' begin at the stripe conductor 41 of the base module 2 are connected to one of the contacts of either SPDT, whereas line 32 and 32' begin at the stripe conductor 42 and are connected to the other contact of either SPDT. Accordingly, depending on the switching state of the changeover switch 38 the double SPDT will connect stripe conductor 41 of the base module 2 straight to the stripe conductor 41' of the extension module 9 and stripe conductor 42 of the base module 2 straight to stripe conductor 42' of the extension module 9, or it will cross connect stripe conductor 41 of the base module 2 to the stripe conductor 42' of the extension module and stripe conductor 42 of the base module 2 to the stripe conductor 41' of the extension module 9. In the exemplary embodiment as depicted in Fig. 5 the straight connection is made by the changeover switch 38.

The interface 3 further comprises a switch and is further connected to the switch 37 for a selectable connection of the supplemental connector 44 of the extension module 9 to the stripe conductor 43'.

Further, by a line set 35 the data communication wiring 5 of the base module 2 is connected to the data communication wiring 5' of the extension module 9. Likewise, by another line set 36 the appliance voltage distribution wiring 6 of the base module 2 is connected to the appliance voltage distribution wiring 6' of the extension module 9. As a result, the extension module 9 is completely connected to the base module 2 and provides additional hybrid connectors 8' in the same manner as the base module 2 to provide its hybrid connectors 8.

Further, similar to the base module 2 having the HF input terminal 22 the extension module 9 is provided with its own HF input terminal 92. In case the extension module 9 is connected to the base module 2 the HF energy supply cable 28 will be placed in the HF input terminal 92 of the extension module 9 rather than in the HF input terminal 22 of the base module 2. By virtue of this, all stripe conductors 41, 42, 43 including the supplemental conductor 44 can be provided with high-frequency power for the electrosurgical instrument 19, and by virtue of the changeover switch 38 and switch 37 the stripe conductors 41, 42, 43 of the base module 2 are being supplied with HF power in a retrograde matter from the extension module 9 by means of the interface 3.

In the configuration as shown in Fig. 5, in the leftmost hybrid connector 8 of the base module 2 a monopolar sub module 7 is inserted and in the next hybrid connector a sub modules 7 providing a neutral electrode is inserted. In the rightmost hybrid connector 8 of the base module 2 a plasma-blend sub modules 7 is inserted. The sub modules 7 placed in the respective hybrid connector 8 are marked in the figures by dithering. Contact established between the sub modules and the conductors 41, 42, 43 (and 44 if applicable) of the HF energy distribution line 4 is marked by a diamond filled with crosshatching, whereas an outlined diamond without any filling symbolizes a contact not being made.

On the extension module 9 the two leftmost hybrid connectors 8' are also being populated by sub modules, one carrying another monopolar sub module 7 and the other carrying a plasma-blend sub module 7. The switch 37 is closed such that the stripe conductors 43' and 44 both provide neutral electrode like the stripe conductor 43. This configuration is termed "plasma-blend, monopolar bridged NE".

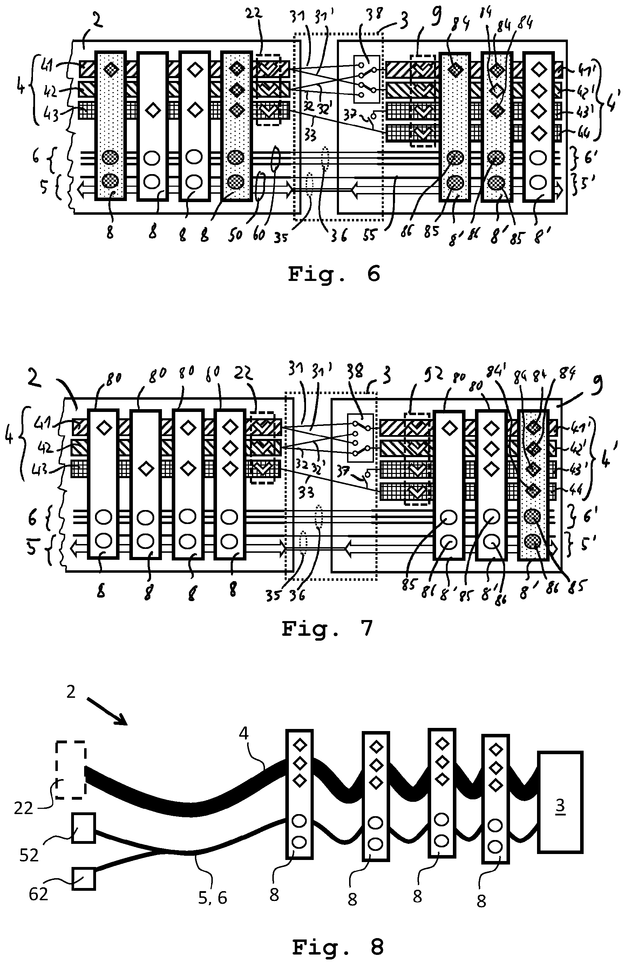

In Fig. 6 a configuration is shown having one monopolar sub module 7 placed in the leftmost hybrid connector of the base module 2 as well as of the extension module 9. Further, in the rightmost hybrid connector 8 of the base module 2 and in the middle hybrid connector 8' of the extension module 9 a universal sub module 7 is placed. However, as opposed to the first configuration shown in Fig. 5, in the second configuration as shown in Fig. 6 the relay 38 is in its other state crossing the lines 31 and 32 such that the stripe conductor 42 of the base module 2 is connected to the stripe conductor 41' of the extension module 9 and the stripe conductor 41 of the base module 2 is connected to stripe conductor 42' of the extension module 9. Thereby the stripe conductors 41, 42 are electrically reversed on the base module 2 to those on the extension module 9. By virtue of this, a configuration featuring a "universal dual activation bipolar with independent neutral electrode" when the switch 37 is open as depicted. (A "dual monopolar" configuration would be achieved if the switch 37 was closed.

In Fig. 7 a third configuration is shown which is significantly different. In this configuration the electrosurgical generator 1 is configured in an ultrasonic configuration for supplying an ultrasonic electrosurgical instrument. No sub module 7 is placed in any of the hybrid connectors 8 of the base module 2, rather an ultrasonic sub module 7 is placed in one of the hybrid connectors 8' of the extension module 9. The changeover switch 38 is in its straight connection state. The switch 37 is in its open state, thereby separating the stripe conductors 43' and 44 of the extension module 9. Accordingly, the HF energy supplied via the HF input terminal 92 including ultrasonic energy to the stripe conductor 44 will be conveyed to the respective contact of the ultrasonic sub module 7 placed in the hybrid connector 8' on the extension module 9. Thereby, a variant for "Ultrasonic Configuration" can be manufactured by placing the corresponding ultrasonic sub module 7 into the hybrid connector 8 of the extension module 9.

For proper activation of the sub modules 7 an enable line 55 is provided on the base module 2 as well as on the extension module 9. This enable line is routed via the connection ports, i.e. the hybrid connectors 8, 8', to a relay 78 on the respective sub modules 7. The relays 78 are configured to convey or to block the HF energy supplied to the respective sub module 7 via the respective hybrid connector 8. The enable line may be utilized to switch on the relay for conveying the HF energy if the sub module 7 is determined to be activated, e.g., by passing a compatibility check. To this end, a microprocessor 24 of the base module 2 queries identification data via the data communication wiring 5 from the respective sub module 7 being placed in the respective hybrid connector 8. The microprocessor 24 co-operates with an activation control unit 25 provided on the base module 2, thereby identifying the sub module configuration and checking validity of the identified configuration against one or more reference configurations stored in a memory 26 that is attached to the activation control unit 25. If a corresponding reference configuration is found then the activation control unit 25 determines that the present sub module configuration is valid and provides a corresponding signal to the microprocessor 24. If validity is established, a corresponding activation signal is transmitted to the respective sub modules 7 found to be valid via the enable line 55, and the relays 78 provided on the respective sub modules 7 will be energized to allow flow of HF energy. Any sub module 7 that is not belonging to the reference configuration will not receive the enable signal via the enable line 55, and accordingly it will not be activated. Thereby integrity of the configuration of the sub modules 7 is verified and maintained. Any "rogue" sub module not conforming to the reference configuration or configurations will thus not be activated. Thereby operational flexibility as well as safety are increased.

The base module 2 may be embodied as a printed circuit board as depicted in Fig. 2A and 2B and Fig. 4 to 7. Thereby a proper support for the connection port, particularly the hybrid connectors 8, is provided and proper mechanical support and stabilization for the sub modules 7 can be achieved. However, such a hard embodiment of the base module 2 is not a necessity. It may also be configured in a soft variant comprising mainly of a cable set for the HF energy and the hybrid connectors 8 as depicted in Fig. 8. Further comprised are the HF energy terminal 22, data terminal 52, and appliance voltage terminal 62 as well as the interface 3 for connection to the extension module 9 (not shown in Fig. 8). This provides for a compact base module 2 that can be manufactured efficiently.

## Claims

1. Electrosurgical generator configured to output HF energy to an electrosurgical instrument (19), comprising an inverter unit (27) generating the HF energy to be output to the electrosurgical instrument (19), and a high-voltage connection supplying the HF energy via at least two electrode lines to at least one output socket (17) configured for connection of the electrosurgical instrument (19), **characterized in that**
the high-voltage connection comprises a base module (2) which is provided with a distribution unit and at least one connection port,
the distribution unit comprising a HF energy distribution line (4) and a data communication wiring (5) for data signals, and being configured to distribute the HF energy supplied from the inverter unit (27) as well as the data signals to the at least one connection port,
said at least one connection port being configured to be connected to a sub module (7) and conveying the HF energy and the data signals to said sub module (7) which supplies the output socket (17) with the HF energy.

2. Electrosurgical generator of claim 1, wherein the at least one connection port is configured as a universal connector to be connectable to sub modules (7) of different types, in particular differing by number and/or kind of electrode lines.

3. Electrosurgical generator of claim 2, wherein the different types of the sub modules (7) comprise a monopolar socket module, a bipolar socket module, a neutral electrode socket module and/or a universal socket module.

4. Electrosurgical generator according to any of the preceding claims, wherein the distribution unit distributes the HF energy and the data signals to a plurality of connection ports.

5. Electrosurgical generator according to any of the preceding claims, wherein the at least one connection port is formed as a hybrid connector (8) comprising a first section (81) for HF energy and a second section (82) for the data signals.

6. Electrosurgical generator according to any of the preceding claims, wherein the HF distribution line (4) comprises high voltage conductors for active electrode, a neutral electrode and at least an additional electrode, preferably a second active electrode.

7. Electrosurgical generator according to the preceding claim, wherein the HF distribution line (4) comprises a conductor for ultrasonic frequency voltage, wherein preferably the conductor for ultrasonic frequency voltage is an additional conductor or is shared with another conductor, further preferably a conductor for a second active electrode.

8. Electrosurgical generator according to any of the preceding claims, wherein the base module (2) further comprises an appliance voltage distribution wiring (6) configured to convey operating power for the sub-module (7) via the connection port, preferably via a third section (83) of the hybrid connector (8).

9. Electrosurgical generator of any of the preceding claims, wherein the base module (2) is a circuit board and the high-frequency distribution line (4) is configured as a high-voltage bus bar (40).

10. Electrosurgical generator of any of the preceding claims, wherein relays (78) are provided for the connection ports, each of the relays (78) interacting with one of the connection ports and being configured to switch on or to block the HF energy conveyed by said one of the connection ports, wherein preferably the relay (78) interacting with one of the connection ports is configured to switch on dependent on a signal issued if the sub module (7) has passed a compatibility check.

11. Electrosurgical generator of the preceding claim, wherein the relay is located on the sub module (7), and preferably the relay (78) is energized by an individual enable signal conveyed by said one of the connection ports connected to said sub module (7).

12. Electrosurgical generator according to any of the preceding claims, wherein an optional extension module (9) is provided which comprises at least one additional connection port (8') and an extended HF distribution line (4') and an extended data communication distribution wiring (5'), wherein the at least one additional connection port (8') is connected to said distribution line (4') and to the data communication distribution wiring (5') like the connection port of the base module (2).

13. Electrosurgical generator according to the preceding claim, wherein the base module (2) comprises an interface (3) for attaching the extension module (9), such that the HF distribution line (4) of the base module (2) is/are connected to the HF distribution line (4') of the extension module (9), and the data distribution wiring (5) and/or appliance voltage distribution wiring (6) of the base module (2) is/are connected to respective distribution wirings of the extension module (9),
wherein preferably the HF distribution line (4') of the extension module (9) is provided with a supplemental conductor (44) for another electrode.

14. Electrosurgical generator according to the preceding claim, wherein the interface (3) further comprises at least one switch (37) configured to isolate or to connect the supplemental conductor (44) of the extension module (9) to one of the conductors (41, 42, 43) of the base module (2), preferably the neutral electrode conductor of the base module (2).

15. Electrosurgical generator according to preceding claims 12 to 14, wherein the interface (3) comprises at least one changeover switch (38) configured to cross-connect electrode conductors of the base module (2) to those of the extension module (9), preferably being actuated automatically depending on the type of the sub module (7) connected to the at least one connection port (8') of the extension module (9),
wherein further preferably the extension board (9) has an own HF input terminal (92) and the base module (2) is supplied with the HF energy via the interface (3).
